# DEMANDE DE BREVET EUROPEEN

(11) **EP 1 632 217 A1**
(43) Date de publication de la demande: **08.03.2006**
(21) Numéro de dépôt: 05291145.0
(22) Date de dépôt: 27.05.2005
(51) Int. Cl.: A61K 8/44, A61K 8/41, A61K 8/34, A61K 8/37, A61Q 5/10

(54) **Composition pour la coloration de fibres kératiniques comprenant un compose polycarboxylique particulier et un coupleur particulier, procédé et dispositif la mettant en oeuvre**

(30) Priorité: 28.05.2004 FR 0405839
(71) Demandeur: L'OREAL, 75008 Paris (FR)
(72) Inventeur: Legrand, Frédéric, 92400 Courbevoie (FR)
(74) Mandataire: Wattremez, Catherine

(57) **Abrégé**

La présente invention a pour objet une composition destinée au traitement des fibres kératiniques humaines, comprenant un composé choisi parmi l'acide éthylènediamine-N,N'-disuccinique, un de ses sels, ou leur mélange, ledit composé étant associé à au moins une base d'oxydation et au moins un coupleur particulier de formule suivante : 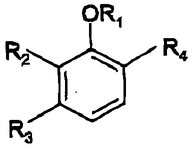 formule dans laquelle R₁ représente un atome d'hydrogène, un radical acyle ; R₂ représente un atome d'halogène, de préférence le chlore, R₃ représente un groupement amino ; R₂ et R₃ pouvant former ensemble, avec les atomes de carbone qui les portent, un noyau benzénique ; R₄ représente un radical alkyle en C₁-C₄, de préférence le radical méthyle ; ou son sel d'addition avec un acide.

Elle concerne de plus un procédé de coloration d'oxydation des fibres kératiniques humaines mettant en oeuvre la composition selon l'invention, ainsi qu'un dispositif à plusieurs compartiments.

## Description

La présente invention a pour objet une composition destinée au traitement des fibres kératiniques humaines, comprenant un composé portant quatre fonctions acide carboxylique, sous forme acide ou sous forme de sel, ledit composé étant associé à au moins un coupleur particulier. Elle concerne de plus un procédé de coloration d'oxydation des fibres kératiniques humaines la mettant en oeuvre, ainsi qu'un dispositif approprié

Il existe plusieurs types de coloration parmi lesquels figurent la coloration permanente (ou coloration d'oxydation) et la coloration directe (coloration semi-permanente) avec ou sans effet d'éclaircissement des fibres.

La coloration d'oxydation met en oeuvre des composés qui sont des précurseurs de colorants d'oxydation, et plus particulièrement une ou plusieurs bases d'oxydation éventuellement associées à un ou plusieurs coupleurs. Ces composés sont des substances incolores ou peu colorées qui, par un processus de condensation oxydative, en présence d'un agent oxydant, conduisent à des composés qui colorent les fibres.

La coloration semi-permanente emploie des colorants directs, qui sont des composés colorants et colorés, et peut être appliquée sur les fibres en présence ou non d'un agent oxydant, selon que l'on souhaite avoir un effet éclaircissant associé à la coloration.

Le domaine de l'invention est plus particulièrement concerné par des procédés de coloration utilisant la combinaison d'au moins une base d'oxydation et d'au moins un coupleur.

Il est en outre à noter que les compositions tinctoriales employées doivent satisfaire à des critères relativement contraignants du fait qu'elles sont appliquées directement sur les fibres et entrent en contact avec la peau, et qu'elles contiennent des ingrédients ou sont utilisées dans des conditions telles (notamment de pH) qui peuvent entraîner des réactions de sensibilisation de la peau, ainsi qu'une dégradation à la longue, des fibres traitées.

Des progrès importants ont été réalisés dans ces divers domaines de traitements des fibres kératiniques. Ainsi, les compositions contiennent de nombreux additifs qui, par exemple, visent à régler la rhéologie de la composition afin qu'elle puisse être appliquée facilement et rapidement, sans couler hors des zones à traiter pendant le temps de pose. Il existe aussi d'autres additifs qui ont pour objectif de limiter ou corriger des effets de dégradation de la fibres kératiniques occasionnés par les procédés.

Cependant, dans certains cas, les additifs présents ne sont pas sans effet sur les résultats mêmes du traitement.

C'est pourquoi on cherche toujours à améliorer la chromaticité, la luminosité, la ténacité des colorations obtenues, tout en conservant une faible sélectivité.

Ainsi, la présente invention a pour objet de proposer des compositions qui résolvent les problèmes ci-dessus.

Un premier objet de l'invention est donc constitué par une composition destinée au traitement de fibres kératiniques humaines, comprenant :
- au moins un composé choisi parmi l'acide éthylènediamine-N,N'-disuccinique, un de ses sels, ou leur mélange,
- au moins une base d'oxydation,
- au moins un coupleur de formule (A) suivante : 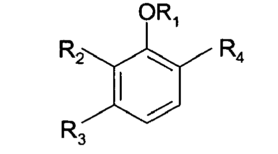 formule dans laquelle :
   R₁ représente un atome d'hydrogène, un radical acyle ;
   R₂ représente un atome d'halogène, de préférence le chlore,
   R₃ représente un groupement amino ; R₂ et R₃ pouvant former ensemble, avec les atomes de carbone qui les portent, former un noyau benzénique ;
   R₄ représente un radical alkyle en C₁-C₄, de préférence le radical méthyle;
ou un sel d'addition avec un acide.

Elle a de même pour objet un procédé de coloration de fibres kératiniques humaines mettant en oeuvre cette composition, ainsi qu'un dispositif à plusieurs compartiments.

Mais d'autres caractéristiques et avantages de la présente invention apparaîtront plus clairement à la lecture de la description et des exemples qui vont suivre.

Dans ce qui va suivre et à moins que le contraire ne soit précisé, les bornes délimitant un domaine de valeurs sont comprises dans ce domaine.

Par ailleurs, les fibres kératiniques humaines traitées conformément à l'invention, sont plus particulièrement des cheveux.

Ainsi que cela a été indiqué auparavant, la composition selon l'invention comprend au moins un composé choisi parmi l'acide éthylènediamine-N,N'-disuccinique, un de ses sels, ou leur mélange.

Les sels sont de préférence des sels de métaux alcalins (comme par exemple le sodium, le potassium), de métaux alcalino-terreux (notamment le calcium, le magnésium), d'ammonium substitués ou non par un à trois radicaux alkyle, identiques ou différents, comprenant 1 à 6 atomes de carbone, de préférence de 1 à 4 atomes de carbone, éventuellement porteurs d'au moins un radical hydroxyle.

De préférence, le composé entrant dans la composition selon l'invention se présente sous la forme d'un sel de sodium.

Conformément à un mode de réalisation particulier de l'invention, la teneur en acide éthylènediamine-N,N'-disuccinique, sous forme acide ou sel, ou en mélange, représente entre 0,01 et 20 % en poids par rapport au poids de la composition, plus particulièrement entre 0,1 et 10 % en poids par rapport au poids de la composition, de préférence entre 0,2 et 5 % en poids par rapport au poids de la composition.

La composition selon l'invention comprend en outre au moins un coupleur de formule (I) suivante: 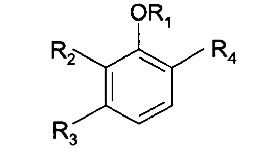 formule dans laquelle :
R₁ représente un atome d'hydrogène, un radical acyle ;
R₂ représente un atome d'halogène, de préférence le chlore,
R₃ représente un groupement amino ; R₂ et R₃ pouvant former ensemble, avec les atomes de carbone qui les portent, former un noyau benzénique ;
R₄ représente un radical alkyle en C₁-C₄, de préférence le radical méthyle;
ou son sel d'addition avec un acide.

Conformément à un premier mode de réalisation de l'invention, le coupleur de formule (A) est le 1-hydroxy-2-méthyl-5-amino-6-chlorobenzène, ou son sel d'addition avec un acide.

Conformément à un deuxième mode de réalisation particulier de l'invention, le coupleur de formule (A) est le 1-acétoxy-2-méthylnaphtalène, ou un sel d'addition avec un acide.

D'une manière générale, les sels d'addition avec un acide les coupleurs de formule (A) sont notamment choisis parmi les chlorhydrates, les bromhydrates, les sulfates, les citrates, les succinates, les tartrates, les lactates, les tosylates, les benzènesulfonates, les phosphates et les acétates.

La composition selon l'invention comprend plus particulièrement une teneur en coupleur de formule (A) comprise entre 0,0001 à 10% en poids par rapport au poids de la composition, de préférence entre 0,005 à 5% en poids par rapport au poids de la composition.

Par ailleurs, la composition selon l'invention comprend au moins une base d'oxydation.

Cette dernière est avantageusement choisie parmi les ortho-phénylènediamines, les para-phénylènediamines, les bis-phénylalkylènediamines, les para-aminophénols, les bis-para-aminophénols, les ortho-aminophénols, les bases hétérocycliques et leurs sels d'addition.

Parmi les para-phénylènediamines, on peut citer à titre d'exemple, la para-phénylènediamine, la para-toluènediamine, la 2-chloro para-phénylènediamine, la 2,3-diméthyl para-phénylènediamine, la 2,6-diméthyl para-phénylènediamine, la 2,6-diéthyl para-phénylènediamine, la 2,5-diméthyl para-phénylènediamine, la N,N-diméthyl para-phénylènediamine, la N,N-diéthyl para-phénylènediamine, la N,N-dipropyl para-phénylènediamine, la N,N-diéthyl 3-méthyl para-phénylènediamine, la N,N-bis-(β-hydroxyéthyl) para-phénylènediamine, la N,N-bis-(β-hydroxyéthyl) 2-méthyl para-phénylènediamine, la N,N-bis-(β-hydroxyéthyl) 2-chloro para-phénylènediamine, la 2-fluoro para-phénylènediamine, la 2-isopropyl para-phénylènediamine, la N-(β-hydroxypropyl) para-phénylènediamine, la 2-hydroxyméthyl para-phénylènediamine, la N,N-diméthyl 3-méthyl para-phénylènediamine, la N, -(éthyl)- N-( β-hydroxyéthyl) para-phénylènediamine, la N-(β,γ-dihydroxypropyl) para-phénylènediamine, la N-(4'-aminophényl) para-phénylènediamine, la N-phényl para-phénylènediamine, la 2-β-hydroxyéthyl para-phénylènediamine, la 2-β-hydroxyéthyloxy para-phénylènediamine, la 2-β-acétylaminoéthyloxy para-phénylènediamine, la N-(β-méthoxyéthyl) para-phénylènediamine, la 4-aminophénylpyrrolidine, la 2-thiényl para-phénylènediamine, le 2-β hydroxyéthylamino 5-amino toluène, la 3-hydroxy 1-(4'-aminophényl)pyrrolidine et leurs sels d'addition avec un acide.

Parmi les para-phénylènediamines citées ci-dessus, la para-phénylènediamine, la para-toluènediamine, la 2-isopropyl para-phénylènediamine, la 2-β-hydroxyéthyloxy para-phénylène-diamine, la 2,6-diméthyl para-phénylènediamine, la 2,6-diéthyl para-phénylènediamine, la 2,3-diméthyl para-phénylènediamine, la 2-chloro para-phénylènediamine, la 2-β-acétylaminoéthyloxy para-phénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition avec un acide.

Parmi les para-aminophénols et les bis-para-aminophénols, on peut citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, le 4-amino 2-chloro phénol, le 4-amino 2,6-dichloro phénol, le 4-amino 6-[(5'-amino 2'-hydroxy 3'-méthyl phényl)méthyl] 2-méthyl phénol, le bis-(5'-amino 2'-hydroxy phényl) méthane et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol ; ainsi que leurs d'addition avec un acide ou avec une base.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2750048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, la 3-amino-5-méthyl-7-imidazolylpropylamino pyrazolo-[1,5-a]-pyrimidine et leurs sels d'addition avec un acide et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Notons que ce qui a été détaillé précédemment sur la nature des sels d'additions des coupleurs de formule (A) est applicable dans le cas des bases d'oxydation mentionnées ci-dessus et l'on pourra s'y référer.

Selon la présente invention, les bases d'oxydation représentent de préférence de 0,0005 à 12% en poids environ du poids total de la composition et encore plus préférentiellement de 0,005 à 8% en poids environ de ce poids.

La composition selon l'invention peut comprendre éventuellement un ou plusieurs coupleurs additionnels différents des coupleurs de formules (A) mentionnés auparavant.

Ces derniers peuvent notamment être choisis parmi ceux classiquement utilisés dans ce domaine, par exemple les méta-aminophénols, les méta-phénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

Ces coupleurs sont plus particulièrement choisis parmi la résorcine, la 2-méthyl résorcine, le méta-aminophénol, le 1-méthyl 2-hydroxy 4β-hydroxyéthylaminobenzène, le 2,4-diamino 1-(β-hydroxyéthyloxy)-benzène, le 2-méthyl-5-amino-phénol, le 5-N-(β-hydroxyéthyl)amino-2-méthyl-phénol, le 3-amino-phénol, le 2-chloro-3-amino-6-méthyl-phénol, le 1,3-dihydroxy-benzène, le 1,3-dihydroxy-2-méthyl-benzène, le 4-chloro-1,3-dihydroxy-benzène, le 2-amino-4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, l'α-naphtol, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

Là encore, ce qui a été détaillé précédemment sur la nature des sels d'additions des coupleurs de formule (A) est applicable dans le cas des coupleurs additionnels mentionnés ci-dessus et l'on pourra s'y référer.

Lorsqu'ils sont présents, ces coupleurs additionnels représentent de préférence de 0,0001 à 10% en poids environ du poids total de la composition, et encore plus préférentiellement de 0,005 à 5% en poids environ.

La composition selon l'invention peut éventuellement comprendre au moins un colorant direct. Celui-ci peut être choisi parmi les espèces cationiques ou non ioniques.

A tire d'exemples non limitatifs, on peut citer les colorants benzéniques nitrés, les colorants azoïques, azométhiniques, méthiniques, tétraazapenthaméthiniques, anthraquinoniques, naphtoquinoniques, benzoquinoniques, phénotiaziniques indigoïdes, xanthéniques, phénanthridiniques, phtalocyanines, ceux dérivés du triarylméthane, seuls ou en mélanges.

Il peut par exemple être choisi parmi les colorants benzéniques nitrés rouges ou orangés suivants :
- le 1-hydroxy-3-nitro-4-N-(γ-hydroxypropyl)amino benzène,
- le N-(β-hydroxyéthyl)amino-3-nitro-4-amino benzène,
- le 1-amino-3-méthyl-4-N-(β-hydroxyéthyl)amino-6-nitro benzène,
- le 1-hydroxy-3-nitro-4-N-(β-hydroxyéthyl)amino benzène,
- le 1,4-diamino-2-nitrobenzène,
- le 1-amino-2-nitro-4-méthylamino benzène,
- la N-(β-hydroxyéthyl)-2-nitro-paraphénylènediamine,
- le 1-amino-2-nitro-4-(β-hydroxyéthyl)amino-5-chloro benzène,
- la 2-nitro-4-amino-diphénylamine,
- le 1-amino-3-nitro-6-hydroxybenzène.
- le 1-(β-aminoéthyl)amino-2-nitro-4-(β-hydroxyéthyloxy) benzène,
- le 1-(β, γ-dihydroxypropyl)oxy-3-nitro-4-(β-hydroxyéthyl)amino benzène,
- le 1-hydroxy-3-nitro-4-aminobenzène,
- le 1-hydroxy-2-amino-4,6-dinitrobenzène,
- le 1-méthoxy-3-nitro-4-(β-hydroxyéthyl)amino benzène,
- la 2-nitro-4'-hydroxydiphénylamine,
- le 1-amino-2-nitro-4-hydroxy-5-méthylbenzène.

Le colorant direct peut aussi être choisi parmi les colorants directs benzéniques nitrés jaunes et jaune-verts, on peut par exemple citer les composés choisis parmi :
- le 1-β-hydroxyéthyloxy-3-méthylamino-4-nitrobenzène,
- le 1-méthylamino-2-nitro-5-(β,γ-dihydroxypropyl)oxy benzène,
- le 1-(β-hydroxyéthyl)amino-2-méthoxy-4-nitrobenzène,
- le 1-(β-aminoéthyl)amino-2-nitro-5-méthoxy-benzène,
- le 1,3-di(β-hydroxyéthyl)amino-4-nitro-6-chlorobenzène,
- le 1-amino-2-nitro-6-méthyl-benzène,
- le 1-(β-hydroxyéthyl)amino-2-hydroxy-4-nitrobenzène,
- la N-(β-hydroxyéthyl)-2-nitro-4-trifluorométhylaniline,
- l'acide 4-(β-hydroxyéthyl)amino-3-nitro-benzènesulfonique,
- l'acide 4-éthylamino-3-nitro-benzoïque,
- le 4-(β-hydroxyéthyl)amino-3-nitro-chlorobenzène,
- le 4-(β-hydroxyéthyl)amino-3-nitro-méthylbenzène,
- le 4-(β,γ-dihydroxypropyl)amino-3-nitro-trifluorométhylbenzène,
- le 1-(β-uréidoéthyl)amino-4-nitrobenzène,
- le 1,3-diamino-4-nitrobenzène,
- le 1-hydroxy-2-amino-5-nitrobenzène,
- le 1-amino-2-[tris(hydroxyméthyl)méthyl]amino-5-nitro-benzène,
- le 1-(β-hydroxyéthyl)amino-2-nitrobenzène,
- le 4-(β-hydroxyéthyl)amino-3-nitrobenzamide.

On peut aussi mentionner les colorants directs benzéniques nitrés bleus ou violets, comme par exemple :
- le 1-(β-hydroxyéthyl)amino-4-N,N-bis-(β-hydroxyéthyl)amino 2-nitrobenzène,
- le 1-(γ-hydroxypropyl)amino 4-N,N-bis-(β-hydroxyéthyl)amino 2-nitrobenzène,
- le 1-(β-hydroxyéthyl)amino 4-(N-méthyl, N-β-hydroxyéthyl)amino 2-nitrobenzène,
- le 1-(β-hydroxyéthyl)amino 4-(N-éthyl, N-β-hydroxyéthyl)amino 2-nitrobenzène,
- le 1-(β,γ-dihydroxypropyl)amino 4-(N-éthyl, N-β-hydroxyéthyl)amino 2-nitrobenzène,
- les 2-nitroparaphénylènediamines de formule suivante : 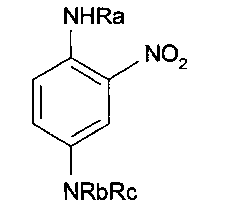
dans laquelle :
- R_{b} représente un radical alkyle en C₁-C₄, un radical β-hydroxyéthyle ou β-hydroxypropyle ou γ-hydroxypropyle ;
- Ra et Rc, identiques ou différents, représentent un radical β-hydroxyéthyle, -β-hydroxypropyle, γ-hydroxypropyle, ou β,γ-dihydroxypropyle, l'un au moins des radicaux R_{b}, R_{c} ou Ra représentant un radical γ-hydroxypropyle et R_{b} et R_{c} ne pouvant désigner simultanément un radical β-hydroxyéthyle lorsque R_{b} est un radical γ-hydroxypropyle, telles que celles décrits dans le brevet français FR 2 692 572.

Le colorant direct peut aussi être choisi parmi les colorants basiques tels que ceux notamment décrits dans le Color Index, 3ème édition, sous les dénominations "Basic Brown 16", "Basic Brown 17", "Basic Yellow 57", "Basic Red 76", "Basic Violet 10", "Basic Blue 26" et "Basic Blue 99", ou des colorants directs acides parmi lesquels on peut plus particulièrement citer les colorants connus dans le Color Index, 3ème édition, sous les dénominations "Acid Orange 7", "Acide Orange 24", "Acid Yellow 36", Acid Red 33", "Acid Red 184", "Acid Black 2", "Acid Violet 43", et "Acid Blue 62", ou encore des colorants directs cationiques tels que ceux décrits dans les demandes de brevet WO 95/01772, WO 95/15144 et EP 714954 et dont le contenu fait partie intégrante de la présente invention.

S'ils sont présents, la teneur en colorant(s) direct(s) dans la composition varie en général de 0,001 à 20% en poids par rapport au poids de la composition, et de préférence de 0,01 à 10% en poids par rapport au poids de la composition.

La composition selon l'invention peut de plus comprendre au moins un tensioactif, de préférence non ionique, anionique, amphotère ou zwittérionique.

Parmi les tensioactifs non ioniques, on peut citer les alcools, les alpha-diols, les alkylphénols polyéthoxylés et/ou polypropoxylés, ayant une chaîne hydrocarbonée comprenant par exemple de 8 à 30 atomes de carbone, de préférence de 8 à 18 atomes de carbone, le nombre de groupements oxyde d'éthylène et/ou oxyde de propylène pouvant aller notamment de 2 à 50.

On peut également citer les copolymères d'oxyde d'éthylène et de propylène, les condensats d'oxyde d'éthylène et/ou de propylène sur des alcools gras ; les amides gras polyéthoxylés ayant de préférence de 2 à 30 moles d'oxyde d'éthylène; les esters d'acides gras de sorbitan oxyéthylénés ayant de 2 à 30 moles d'oxyde d'éthylène ; les esters d'acides gras de sucrose, les esters d'acides gras et de polyéthylèneglycol, les alkylpolyglycosides, les dérivés de N-alkyl glucamine, etc.

Parmi les tensioactifs anioniques, on peut citer entre autres les sels (en particulier les sels alcalins, notamment de sodium, de magnésium, les sels d'ammonium, les sels d'amines, les sels d'aminoalcools) d'alkylsulfates, alkyléthersulfates, alkylamidoéthersulfates, alkylarylpolyéthersulfates, monoglycérides sulfates ; alkylsulfonates, alkylphosphates, alkylamidesulfonates, alkylarylsulfonates, α-oléfine-sulfonates, paraffine-sulfonates ; alkyl(C₆-C₂₄) sulfosuccinates, alkyl(C₆-C₂₄) éthersulfosuccinates, alkyl(C₆-C₂₄) amidesulfosuccinates ; alkyl(C₆-C₂₄) sulfoacétates ; acyl(C₆-C₂₄) sarcosinates et glutamates ; esters d'alkyl(C₆-C₂₄)polyglycosides carboxyliques ; alkylsulfosuccinamates ; acyliséthionates et N-acyltaurates ; le radical alkyle ou acyle de tous ces différents composés comportant de préférence de 12 à 20 atomes de carbone, et le radical aryle désignant de préférence un groupement phényle ou benzyle.

Conviennent aussi les sels d'acides gras tels que les sels des acides oléique, ricinoléique, palmitique, stéarique, les acides d'huile de coprah hydrogénée ou non ; les acides d'alkyl D-galactoside uroniques et leurs sels, les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'alkylène en particulier d'éthylène, et leurs mélanges.

En ce qui concerne les tensioactifs amphotères ou zwittérioniques conviennent notamment les dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer amphocarboxyglycinates et amphocarboxypropionates, comme par exemple les disodium cocoamphodiacetate, disodium lauroamphodiacetate, disodium caprylamphodiacetate, disodium cocoamphodipropionate, disodium lauroamphodipropionate, etc. (produits de la gamme Miranol®, notamment Miranol® C2M concentré commercialisés la société Rhodia Chimie).

La teneur en tensioactif dans la composition représente habituellement de 0,01 à 40 % en poids et de préférence de 0,5 à 30 % en poids, du poids total de la composition.

La composition conforme à l'invention peut renfermer au moins un agent épaississant, associatif ou non. Ils sont choisis de préférence parmi les dérivés de cellulose, comme par exemple les hydroxypropyl ou carboxyméthyl celluloses ; les dérivés de guar ; les gommes d'origine microbienne, comme les gommes de scléroglucane, de xanthane ; les gommes issues d'exudats végétaux telles que les gommes arabiques, Ghatti, Karaya, Tragacanthe ; ainsi que les épaississants synthétiques comme les homopolymères d'acide acrylique, d'acrylate, d'acrylamide, d'acide 2-acrylamido-2-méthyl-propane sulfonique, réticulés ou non ; les pectines ; les alginates.

La composition peut aussi comprendre un ou plusieurs polymères associatifs en tant qu'épaississant comme notamment, les dérivés de polyuréthanes associatifs, les dérivés de celluloses associatifs, les dérivés de polyvinyllactames associatifs et les dérivés de polyacides insaturés associatifs, et leurs mélanges.

Habituellement, au cas où il est présent, la teneur en polymère épaississant, associatif ou non, représente de 0,01 et 10% en poids par rapport au poids de la composition, et en particulier entre 0,1 et 5% en poids par rapport au poids de la composition.

La composition conforme à l'invention peut également comprendre divers adjuvants utilisés classiquement, tels que des polymères substantifs cationiques ou amphotères, des polymères anioniques, cationiques, non ioniques, amphotères, zwittérioniques, des agents épaississants minéraux, des agents antioxydants, des acides alpha-oxocarboxyliques (par exemple l'acide oxalique, l'acide glyoxalique, l'acide pyruvique ou l'acide alpha-cétoglutarique), des agents de pénétration, des parfums, des tampons, des acides aminés basiques comme l'arginine notamment, des agents dispersants, des agents peptisants, des agents de conditionnement tels que par exemple des cations, des silicones volatiles ou non volatiles, modifiées ou non modifiées, des agents filmogènes, des céramides, des vitamines ou provitamines, des agents conservateurs, des agents stabilisants, des agents opacifiants ou matifiants comme le dioxyde de titane, des charges minérales, comme les argiles, les silices notamment pyrogénées à caractère hydrophile ou hydrophobe, des polymères liants tels que la vinylpyrrolidone, des lubrifiants comme les stéarates, des agents de contrôle du dégagement d'oxygène tels que le carbonate ou l'oxyde de magnésium, les filtres solaires, etc.

Les adjuvants cités ci-dessus sont en général présents en quantité comprise pour chacun d'eux entre 0,01 et 20% en poids par rapport au poids de la composition.

Le milieu de la composition est un milieu cosmétiquement acceptable.

Il est de préférence constitué par un milieu aqueux comprenant par de l'eau et éventuellement un ou plusieurs solvants organiques acceptables sur le plan cosmétique.

Plus particulièrement, les solvants organiques sont choisis parmi les monoalcools ou les diols, linéaires ou ramifiés, de préférence saturés, comprenant 2 à 10 atomes de carbone, tels que l'alcool éthylique, l'alcool isopropylique, l'hexylèneglycol (2-méthyl 2,4-pentanediol), le néopentylglycol et le 3-méthyl-1,5-pentanediol ; les alcools aromatiques tels que l'alcool benzylique, l'alcool phényléthylique ; les glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ; ainsi que les alkyléthers de diéthylèneglycol, notamment en C₁-C₄, comme par exemple, le monoéthyléther ou le monobutyléther du diéthylèneglycol, seuls ou en mélange.

Les solvants usuels décrits ci-dessus, s'ils sont présents, représentent habituellement de 1 à 40 % en poids, plus préférentiellement de 5 à 30 % en poids, par rapport au poids total de la composition.

Le pH de la composition selon l'invention est généralement compris entre 3 et 12 environ, et de préférence entre 5 et 11 environ.

Il peut être ajusté à la valeur désirée au moyen d'agents acidifiants ou alcalinisants.

Parmi les agents acidifiants, on peut citer, à titre d'exemples, les acides minéraux ou organiques comme l'acide chlorhydrique, l'acide orthophosphorique, l'acide sulfurique, l'acide acétique.

Parmi les agents alcalinisants on peut citer, à titre d'exemples, l'ammoniaque, les carbonates alcalins, les alcanolamines telles que les mono-, di- et triéthanolamines ainsi que leurs dérivés, les hydroxydes de sodium ou de potassium et les composés de formule suivante : 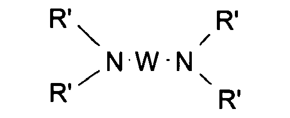 dans laquelle W est un reste propylène éventuellement substitué par un groupement hydroxyle ou un radical alkyle en C₁-C₆ ; les radicaux R', identiques ou différents, représentent un atome d'hydrogène, un radical alkyle en C₁-C₆ ou hydroxyalkyle en C₁-C₆.

La composition selon l'invention peut en outre comprendre au moins un agent oxydant. On parle dans ce cas de composition prête à l'emploi.

Par composition prête à l'emploi, on entend au sens de la présente invention, la composition destinée à être appliquée immédiatement sur les fibres kératiniques, c'est à dire qu'elle peut être stockée telle quelle avant utilisation ou résulter du mélange extemporané de deux ou plusieurs compositions.

Selon une première variante, ladite composition est obtenue en mélangeant la composition selon l'invention avec une composition oxydante.

Selon une autre variante, la composition prête à l'emploi est obtenue en mélangeant une composition comprenant au moins au moins une base d'oxydation telle qu'elles ont été définies auparavant, avec au moins une composition oxydante ; le composé portant les quatre fonctions carboxyliques pouvant être présent soit dans la première composition, soit dans la seconde, soit dans les deux.

Habituellement, l'agent oxydant présent dans la composition oxydante précitée est choisi parmi le peroxyde d'hydrogène, les peroxydes de métaux alcalins ou alcalino-terreux, comme le sodium, le potassium, le magnésium ; le peroxyde d'urée, les bromates ou ferricyanures de métaux alcalins, les persels tels que les perborates et les persulfates de métaux alcalins ou alcalino-terreux, comme le sodium, le potassium, le magnésium, seuls ou en mélanges.

La teneur en agent oxydant est en général comprise entre 1 et 40 % en poids, par rapport au poids de la composition prêt à l'emploi de préférence entre 1 et 20 % en poids par rapport au poids de la composition prêt à l'emploi.

Généralement, la composition oxydante utilisée est une composition aqueuse et peut se trouver sous la forme d'une solution ou encore d'une émulsion.

Habituellement, on mélange la composition exempte d'agent oxydant avec environ 0,5 à 10 équivalents en poids de la composition oxydante.

Notons que le pH de la composition prête à l'emploi est plus particulièrement compris entre 4 et 12, de préférence entre 7 et 11,5.

Le pH de la composition peut être réglé au moyen d'un agent alcalinisant ou acidifiant notamment choisis parmi ceux mentionnés auparavant, dans le cadre de la description selon l'invention.

La présente invention a de plus pour objet un procédé mettant en oeuvre la composition selon l'invention.

Plus particulièrement, le procédé consiste à appliquer sur des fibres kératiniques humaines, sèches ou humides, la composition qui vient d'être décrite pendant une durée suffisante pour obtenir l'effet souhaité.

La composition prête à l'emploi appliquée sur les fibres est obtenue comme indiqué ci-dessus.

La composition prête à l'emploi est donc appliquée sur les fibres kératiniques, sèches ou humides, puis laissée pendant un temps de pose suffisant pour obtenir la coloration recherchée.

Le temps de pose est en général compris entre quelques secondes et 30 minutes, de préférence entre 3 et 15 minutes.

La température à laquelle la composition est laissée agir, est en général comprise entre la température ambiante (15 à 25°C) et 220°C, plus particulièrement entre la température ambiante et 80°C, de préférence entre 15 et 40 °C.

A l'issue de cette période, la composition est éliminée par un rinçage à l'eau, suivi d'un lavage avec un shampooing, puis éventuellement d'un séchage.

La présente invention concerne de plus un dispositif à deux compartiments pour la mise en oeuvre du procédé qui vient d'être décrit. Ce dispositif comprend au moins un compartiment renfermant la composition selon l'invention, et au moins un compartiment renfermant un agent oxydant.

Les exemples qui suivent illustrent la présente invention sans toutefois en limiter la portée.

### EXEMPLES

On a préparé les compositions colorantes suivantes (exprimée en grammes) :

| **Dénomination Matières Premières** | **Compositions** | |
|---|---|---|
| | **A** | **B** |
| Alcools gras oxyéthylénés | 32,5 | 32,5 |
| Acide oléique | 2 | 2 |
| Alcool oléique | 1,8 | 1,8 |
| Amide gras | 4 | 4 |
| Glycérine | 3 | 3 |
| Polycondensat de polyéthylèneglycol à 150 ou 180 moles d'oxyde d'éthylène, d'alcool décylique et de méthylène bis(4-cyclohexylisocyanate) (SMDI), à 35% en poids dans un mélange de propylèneglycol (39%) et d'eau (26%) (Aculyn 44® ; Société Rohm & Haas) | 2 | 2 |
| Copolymère chlorure de diméthyl diallyl ammonium / acide acrylique (80/20) en solution aqueuse protégée (Merquat 280 ® ; Société Nalco) | 2 | 2 |
| Acide éthylènediamine disuccinique, sel trisodique en solution aqueuse (Octaquest E30® ; Société Octel) | 0,8 | 1,8 |
| Réducteur | qs | qs |
| Ammoniaque (20% NH₃) | 8 | 8 |
| 1-hydroxy-4-aminobenzène | / | 0,545 |
| 1-(2-hydroxyéthyl)-4,5-diaminopyrazole | 1,075 | / |
| 1-hydroxy-2-méthyl-5-amino-6-chlorobenzène | 0,788 | / |
| 1-acétoxy-2-méthylnaphthalène | / | 1,001 |
| Eau | qsp 100g | |

Les compositions colorantes ont été mélangées, au moment de l'emploi, dans un bol en plastique et pendant 2 minutes, à une composition aqueuse oxydante à 6 % en eau oxygénée, à raison de 1 partie de composition colorante pour 1,5 parties de composition oxydante.

On a appliqué les mélanges obtenus sur des mèches de cheveux naturels à 90% de blancs et on a laissé poser 30 minutes.

On a ensuite rincé les mèches à l'eau, on les a lavées au shampooing standard, à nouveau rincées à l'eau, puis séchées et démêlées.

Les cheveux ont été teints :
- dans une nuance rouge puissante pour la composition colorante prête à l'emploi issue de la composition A
- dans une nuance cuivrée - rouge puissante pour la composition colorante prête à l'emploi issue de la composition B.
De plus, les cheveux ne sont pas rêches.

## Revendications

1. Composition destinée au traitement de fibres kératiniques humaines, comprenant :
- au moins un composé choisi parmi l'acide éthylènediamine-N,N'-disuccinique, un de ses sels, ou leur mélange.
- au moins une base d'oxydation,
- au moins un coupleur de formule (A) suivante : 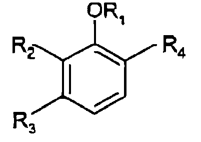 formule dans laquelle :
R₁ représente un atome d'hydrogène, un radical acyle;
R₂ représente un atome d'halogène, de préférence le chlore.
R₃ représente un groupement amino ; R₂ et R₃ pouvant former ensemble, avec les atomes de carbone qui les portent, former un noyau benzénique ;
R₄ représente un radical alkyle en C₁-C₄, de préférence le radical méthyle;
ou son sel d'addition avec un acide.

2. Composition selon la revendication précédente, **caractérisée en ce que** la teneur en composé est comprise entre 0,01 et 20 % en poids par rapport au poids de la composition, plus particulièrement entre 0,1 et 10 % en poids par rapport au poids de la composition, de préférence entre 0,2 et 5 % en poids par rapport au poids de la composition.

3. Composition selon l'une quelconque des revendications précédentes, **caractérisée en ce que** le coupleur de formule (A) est le 1-hydrvxy-2-méthyl-5-amino-6-chlorobenzène, ou son sel d'addition avec un acide.

4. Composition selon l'une quelconque des revendications précédentes, **caractérisée en ce que** le coupleur de formule (A) est le 1-acétoxy-2-méthylnaphtalène, ou un sel d'addition avec un acide.

5. Composition selon l'une quelconque des revendications précédentes, **caractérisée en ce que** la teneur en coupleur de formule (A) représente de 0,0001 à 10% en poids par rapport au poids de la composition, de préférence de 0,005 à 5% en poids par rapport au poids de la composition.

6. Composition selon l'une quelconque des revendications précédentes, **caractérisée en ce que** la base d'oxydation est choisie parmi les ortho-phénylènediamines, les paraphénylènediamines, les bis-phénylalkylènediamines, les para-aminophénals, les bis-para-aminophénols, les ortho-aminophénols, les bases hétérocycliques et leurs sels d'addition avec un acide, ainsi que leurs mélanges.

7. Composition selon l'une quelconque des revendications précédentes, **caractérisée en ce que** la teneur en base d'oxydation représente de 0,0005 à 12% en poids par rapport au poids de la composition, de préférence de 0,005 à 8% en poids par rapport au poids de la composition.

8. Composition selon l'une quelconque des revendications précédentes, **caractérisée en ce que** la composition comprend au moins un coupleur additionnel, différent du coupleur de formule (A), choisi parmi les méta-aminophénols, les métaphénylènediamines, les méta-diphénols, les naphtols, les coupleurs hétérocycliques, leurs sels d'addition avec un acide, ainsi que leurs mélanges.

9. Composition selon la revendication précédente, **caractérisée en ce que** la teneur en coupleur additionnel représente de 0,0001 à 10% en poids par rapport au poids de la composition, de préférence de 0,005 à 5% en poids par rapport au poids de la composition.

10. Composition selon l'une quelconque des revendications précédentes, **caractérisée en ce qu'**elle comprend au moins un colorant direct, cationique ou non ionique, choisi parmi les colorants benzéniques nitrés, les colorants azoïques, azométhiniques, méthiniques, tétraazapenthaméthiniques, anthraquinoniques, naphtoquinoniques, benzo-quinoniques, phénotiaziniques indigoïdes, xanthéniques, phénanthridiniques, phtalocyanines, ceux dérivés du triarylméthane, ainsi que les colorants naturels, seuls ou en mélanges.

11. Composition selon la revendication précédente, **caractérisée en ce que** la teneur en colorant direct varie de 0,001 à 20% en poids par rapport à la composition, et de préférence de 0,01 à 10% du poids total de la composition.

12. Composition selon l'une quelconque des revendications précédentes, **caractérisée en ce qu'**elle comprend au moins un agent oxydant.

13. Composition selon la revendication précédente, **caractérisée en ce que** l'agent oxydant est choisi parmi le peroxyde d'hydrogène, le peroxyde d'urée, les bromates ou ferricyanures de métaux alcalins, les persels tels que les perborates et les persulfates, seuls ou en mélanges.

14. Procédé de coloration des fibres kératiniques humaines consistant à mettre en contact une composition selon l'une quelconque des revendications précédentes, avec lesdites fibres, sèches ou humides, pendant une durée suffisante pour obtenir l'effet souhaité.

15. Dispositif à deux compartiments pour la mise en oeuvre du procédé selon la revendication précédente, **caractérisée en ce qu'**il comprend au moins un compartiment renfermant la composition selon l'une quelconque des revendications 1 à 11, et au moins un compartiment renfermant un agent oxydant.
